# EUROPEAN PATENT APPLICATION

(11) **EP 4 023 644 A1**
(43) Date of publication of application: **06.07.2022**
(21) Application number: 20461611.4
(22) Date of filing: 30.12.2020
(51) Int. Cl.: C07D 407/12

(54) **PROCESS FOR THE PREPARATION OF A PHARMACEUTICAL AGENT**

(71) Applicant: Zaklady Farmaceutyczne Polpharma SA, 83-200 Starogard Gdanski (PL)
(72) Inventor: PUNDA, Pawel, 80-180 Gdansk (PL); RITZEN, Bas, 6416 GE Heerlen (NL); DE LANGE, Ben, 6151 GE Munstergeleen (NL)

(57) **Abstract**

This invention relates to a process for the preparation of empagliflozin comprising lithiation of (3S)-3-[4-(5-bromo-2-chlorobenzyl)phenoxy]tetrahydrofuran using lithium reagent, coupling the lithiated anion species with (3R,4S,5R,6R)-3,4,5-tris[(trimethylsilyl)oxy]-6-{[(trimethylsilyl)oxy]methyl}tetrahydro-2H-pyran-2-one and reducing the product methyl 1-C-(4-chloro-3-{4-[(3S)-tetrahydrofuran-3-yloxy]benzyl}phenyl)-α-D-glucopyranoside] using triethylsilane and boron trifluoride diethyl etherate and optionally purifying the crude product, wherein the reduction is conducted in the presence of molecular sieves.

## Description

The present invention relates to a process for the preparation of a pharmaceutical agent, and particularly to a process for the preparation of empagliflozin.

Empagliflozin is named (1S)-1,5-anhydro-1-(4-chloro-3-{4-[(3S)-tetrahydrofuran-3-yloxy]benzyl}-phenyl)-D-glucitol and has the following structure:

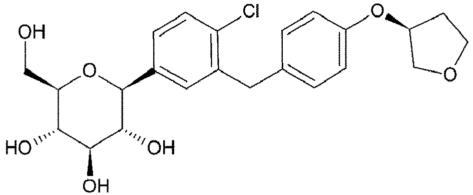

Empagliflozin is an antidiabetic agent from the group of sodium glucose co-transporter 2 (SGLT2) inhibitors for the treatment of type 2 diabetes mellitus.

Empagliflozin is marketed as Jardiance in the form of an immediate-release tablet for once-daily oral administration at a recommended dose of 25 mg as an adjunct to diet and exercise to improve glycaemic control in adults with type 2 diabetes mellitus either as mono-therapy or as an add-on with other oral antidiabetic treatments or insulin.

Empagliflozin and a process for its preparation, was first described in WO2005/092877 and the disclosed process is schematically represented as follows:

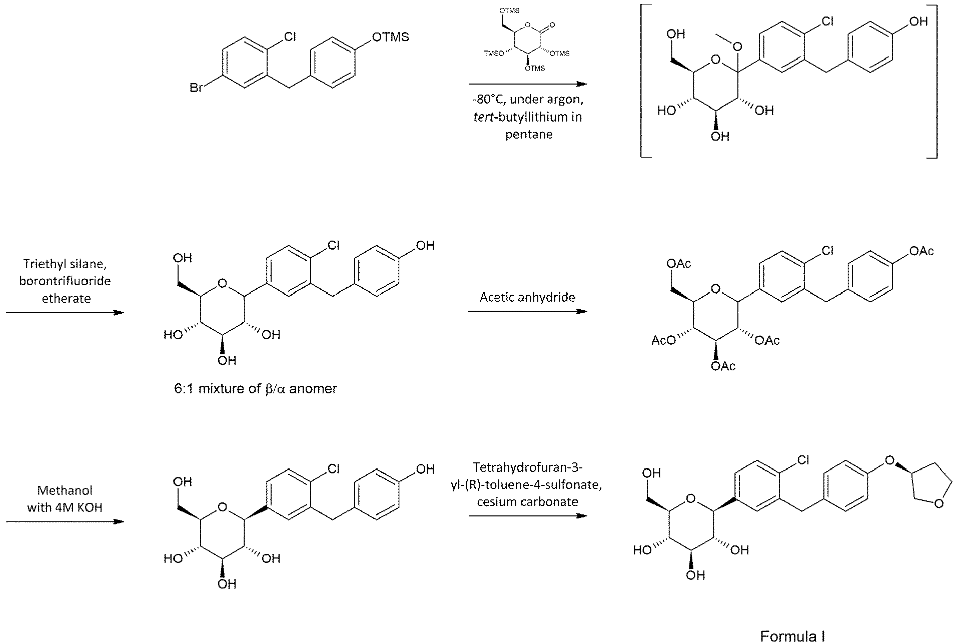

The process disclosed in WO2005/092877 has several disadvantages, such as
a) involvement of extensive chromatography purification steps to isolate the intermediate compound of Formula (e) and the final compound,
b) obtaining the intermediate of Formula (e) as a 6:1 mixture of β/α anomer, which requires additional purification steps such as formation of acetyl derivative followed by neutralization of the same to get pure β-anomer, and
c) isolation of final empagliflozin by chromatography using dichloromethane/methanol.

The document WO2006/120208 discloses processes for the preparation of empagliflozin through the intermediate of 4-(iodo/bromo)-1-chloro-2-(4-tetrahydrofuran-3-yloxy-benzyl)benzene. In this process, the hydroxyl groups of the gluconolactone moiety are protected with trimethylsilyl groups. The process discloses the reaction where after the C-C bond formation the resultant intermediate is deprotected and the hemiketal is formed in the presence of methanesulphonic acid. Due to the presence of different by-products the crude product is purified in multiple steps including extractions, protection with acetyl group followed by isolation of the acetylated intermediate, deprotection and final recrystallization. Another embodiment discloses purification of the crude product by extraction with ethyl acetate and column chromatography. Also WO2018/207111 discloses the same elaborate purification of the final product.

In Org. Lett. 2014, 16, 4090-4093 the synthesis of empagliflozin is described. Regarding the reduction conditions of methyl 1-C-(4-chloro-3-{4-[(3S)-tetrahydrofuran-3-yloxy]benzyl}phenyl)-α-D-glucopyranoside the authors stated that the reduction reaction in the presence of boron trifluoride diethyl etherate is sensitive to the presence of water, i.e. water promotes the side reaction leading to formation of furanosides. They proposed to use aluminum trichloride as a Lewis acid to mediate the reduction. This reagent has been found to be much more preferred than boron trifluoride diethyl etherate for practical reasons. AlCl₃ gave comparable results and the reaction carried out in its presence was less sensitive to the presence of water.

However, the use of aluminum trichloride, even in an organic solvent system (methylene chloride/acetonitrile), reveals some drawbacks. Aluminum trichloride imparts a colloidal consistency to the reaction mixture, which makes it difficult to work up, especially after quenching with water. Furthermore, quenching of the reaction with water is an exothermic process and requires careful temperature control. The above technological disadvantages do not occur when using boron trifluoride diethyl etherate, but the problem of side reaction in the presence of water remains.

As it could be concluded from the above, preparation of empagliflozin using prior art processes suffers from many drawbacks with regards to purity and/or necessity to use non-convenient reactants. Preparation of empagliflozin with an acceptable yield and purity requires numerous steps and isolation of multiple intermediates prior to the isolation of the final product. Consequently, further optimisation of the process is desirable to improve efficiency and economy of the process.

Indeed, both large-scale chemical syntheses and purifications are subjected to constant review and optimisation. Owing to economies of scale, any small improvement in a given reaction or purification parameter is particularly economically significant. Therefore, optimisation of a large-scale synthesis or purification that provides, for example, an increase in chemical yield, decrease in step-to-step manipulation, decrease in reaction time, decrease in reaction temperature, decrease in amount of catalyst or solvent used, increase in the favourability of reagents, reduction in side-product formation, a more environmentally benign synthesis or increase in chemical purity is of interest both to chemical manufacturers and suppliers.

Moreover, step optimisation that reduces the need for multiple or hard-to-perform purifications are particularly beneficial. Any improvement in the ease of purification or isolation, through telescoping (wherein two or more, previously independent synthetic transformations converge into a "single" process and therefore require only one purification step) of synthetic procedures, or the identification of an intermediate for recrystallisation, or precipitation, or removal of impurities through conversion into a transient intermediate, or substitution for a cheaper, more environmentally friendly or less toxic reagent, provide an attractive and economically desirable goal. In situations where more traditional purification procedures yield poor or unsuitable results, it becomes necessary that more innovative solutions are required.

However, practical achievement of any such improvement is not straightforward, and even careful optimisation of individual parameters of a synthetic or purification step will often fail to provide a workable advantage within an overall production process.

Although processes to prepare empagliflozin are established, there remains a need in the art for improving these processes, in particular with respect to reducing the number of preparation/purification steps, reducing the number of isolated intermediates and increasing the overall yield and purity of the final product.

The object of the present invention is to provide an advantageous process for preparing empagliflozin, in particular a process that leads to reduced side reactions that impact the yield and the impurity profile of the substance obtained by the process.

In particular, an object of the present invention is to provide a process in which unwanted side reactions are reduced by carrying out the reduction reaction in the presence of molecular sieves. Preferably, the C-C bond forming step, i.e., the coupling step, which precedes the reduction is carried out as a continuous flow process.

It has been found that the process provides empagliflozin with improved yield and purity. By using molecular sieves it was possible to reduce the amount of critical impurities, (1S)-1,4-anhydro-1-(4-chloro-3-{4-[(3S)-tetrahydrofuran-3-yloxy]benzyl}phenyl)-D-glucitol (impurity 1) and (1R)-1,4-anhydro-1-(4-chloro-3-{4-[(3S)-tetrahydrofuran-3-yloxy]benzyl}phenyl)-D-glucitol (impurity 2). Moreover, when performing the lithiation as a continuous flow process it was possible to further reduce the amount of the two mentioned impurities.

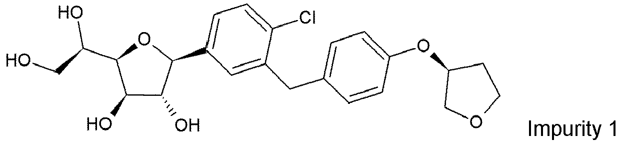

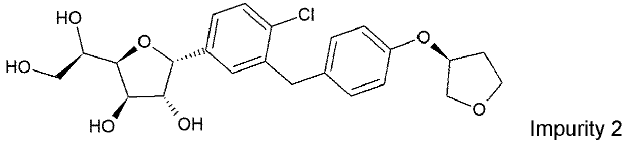

In a first aspect, the invention provides a process for the preparation of empagliflozin (Formula I) comprising lithiation of (3S)-3-[4-(5-bromo-2-chlorobenzyl)phenoxy]tetrahydrofuran (Formula II) using a lithium reagent, coupling the lithiated anion species with (3R,4S,5R,6R)-3,4,5-tris[(trimethylsilyl)oxy]-6-{[(trimethylsilyl)oxy]methyl}tetrahydro-2H-pyran-2-one (Formula IV), reducing the obtained intermediate methyl 1-C-(4-chloro-3-{4-[(3S)-tetrahydrofuran-3-yloxy]benzyl}phenyl)-α-D-glucopyranoside (Formula V) using triethylsilane and boron trifluoride diethyl etherate and optionally purifying the crude product by crystallization, wherein the reduction is conducted in the presence of molecular sieves.

Accordingly, empagliflozin can be prepared according to following synthetic scheme:

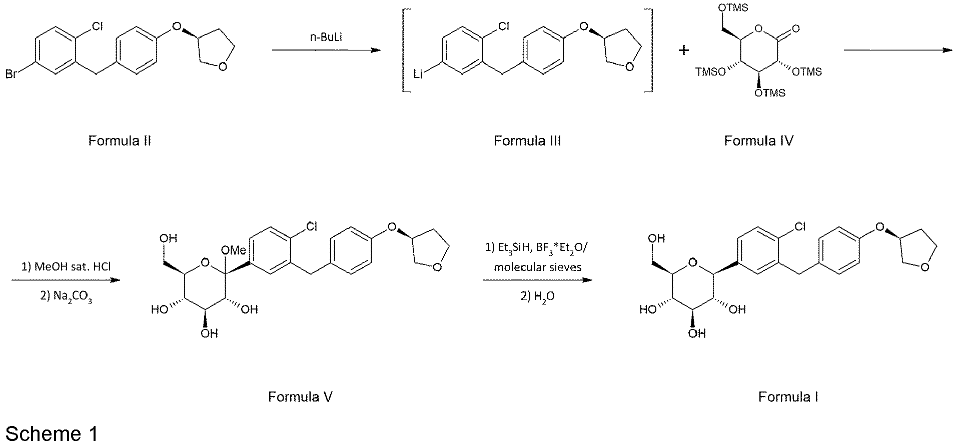

The synthesis starts with lithiation of compound of Formula II with butyllithium to form a lithium salt (Formula II) which is reacted with gluconolactone of Formula IV. The reaction mixture is quenched with methanol saturated with HCI. The product having the Formula V is then reduced with triethylsilane and boron trifluoride diethyl etherate in the presence of molecular sieves. Then water is added to the reaction mixture and the crude product is filtered and recrystallized to yield pure empagliflozin.

The inventors solved the problems with side reactions during the reduction step resulting from the presence of water by adding molecular sieves in order to decrease the water content in the reduction mixture to KF < 0.20%. It is known that under certain conditions zeolites, including molecular sieves, may catalyze reactions of sugar moieties, e.g. hydrolysis, isomerization and dehydration (A. P. Rauter et al., Advances in Carbohydrate Chemistry and Biochemistry, vol. 63 (2010), p. 30-83). Using zeolites may therefore increase the risk of formation of undesired by-products. Experiments of the inventors of the present invention surprisingly showed that purity of empagliflozin obtained in the presence of molecular sieves is higher than of the analogous product obtained in absence of the molecular sieves.

In one aspect the invention relates to the reduction of the compound of Formula V to crude empagliflozin using triethylsilane and boron trifluoride diethyl etherate in the presence of molecular sieves. Solvents suitable for this reaction are halogenated alkanes, organic nitriles, tetrahydrofuran, 2-methyltetrahydrofurane, tetrahydropyran, methyltetrahydropyran, pyridine, 1,4-dioxane, toluene, chlorobenzene, nitrobenzene and anisole or mixtures thereof. Halogenated alkanes include dichloromethane, chloroform, carbon tetrachloride, dichloroethane, trichloroethane and tetrachloroethane. Organic nitriles include acetonitrile, propionitrile, butyronitrile and higher analogues. Preferably, a mixture of dichloromethane and acetonitrile is used. More preferably, the ratio of dichloromethane to acetonitrile is 1:2 (v/v).

The triethylsilane solution is usually cooled down to a temperature in the range from 10 to -30°C, preferably, in the range from 5 to -20°C, under inert atmosphere and then boron trifluoride diethyl etherate is added while stirring. In the next step the compound of Formula V is added to the solution. After quenching of the reduction mixture, the molecular sieves are filtered off.

By conducting the reduction in the presence of molecular sieves it was possible to significantly decrease the amount of critical impurities 1 and 2 by 0.07% (UPLC) (Table 1).

Molecular sieves that can be used in the reduction reaction according to the present invention are basically any type of molecular sieves. Preferably, sieves with pore size of 3 or 4 Å are used. Before being used, molecular sieves are activated. The activation can be realized by various methods known in the art, for example by vacuum drying at a pressure of 50 mbar and a temperature of 150°C, or by air drying at a temperature of 190°C.

According to the present invention, the reduction of the compound of Formula V to crude empagliflozin is carried out using triethylsilane and boron trifluoride diethyl etherate in the presence of molecular sieves. By "carrying out the reduction of the compound of Formula V in the presence of molecular sieves" it is meant the reduction reaction and a cumulative mass of molecular sieves used both in the reduction mixture where the reducing agent is present and in the solution of the compound of Formula V to be added, if sieves are used for preliminary removal of water from that solution.

The amount of molecular sieves used for the reduction is in the range of 5 to 50% w/w based on the mass of the compound of Formula V used for the reduction. Preferably, the amount of molecular sieves used in the reduction is approximately 5-20% w/w based on the mass of the compound of Formula V used for the reduction.

In one embodiment of the invention, the total amount of molecular sieves is present in the reduction mixture.

In another embodiment molecular sieves are present both in the reduction mixture and in the solution of the compound of Formula V to be reduced. Preferably, the amount of molecular sieves used for preliminary removal of water in the solution of compound of Formula V prior to adding it to the solution of the reducing agent, is in the range of 5 to 50% w/w of the mass of the compound of Formula V used for the reduction. Preferably, the amount of molecular sieves in the reduction mixture is approximately 5-20% w/w of the mass of the compound of Formula V to be reduced.

In a more preferred embodiment, the amount of molecular sieves used in the reduction mixture is 10% w/w and in the solution of the compound of Formula V it is 10% w/w, based on the mass of the compound of Formula V subjected to reduction.

After reacting compound of Formula V with boron trifluoride diethyl etherate and triethylsilane, the reaction mixture is quenched with water and crude empagliflozin is isolated.

Optionally, crude empagliflozin is purified by recrystallization. Suitable solvents are lower alcohols, for example methanol, ethanol, propanol. Preferably, 2-propanol is used.

The lithiation reaction of compound of Formula II and the following coupling reaction of compound of Formula III with compound of Formula IV in order to obtain compound of Formula V (Scheme 1) can be performed as a batch process or as a flow process. Both processes give good results in terms of purity of the end product. However, the advantage of using the flow process is safer handling of lithium reactants and less by-products. Moreover, when the compound of Formula V is obtained in a continuous flow process, the yield and purity are higher than for the batch process (Table 1). When comparing the batch process the flow process allows for a reduction of total impurities to the level of approximately 0.2% - from total impurities of about 0.6% to 0.4% as in the batch process.

In the first embodiment, the lithiation and coupling reactions are performed as a batch process. Preferably the lithiation and the following coupling are conducted in a nonpolar or medium polar environment. Suitable solvents include tetrahydrofurane, 2-methyltetrahydrofurane, dioxane, diethyl ether, dibutyl ether, diisopropyl ether, cyclopentyl methyl ether, methyl tert-butyl ether, toluene, pentane, hexane, and cyclohexane or mixtures thereof. Preferred mixtures comprise mixtures of tetrahydrofurane or 2-methyltetrahydrofurane with toluene or hexane or cyclohexane. In a preferred embodiment a mixture of tetrahydrofurane or 2-methyltetrahydrofurane with toluene is used, preferably a 1:3 mixture.

Lithiating agents which can be used are, for example, tert-butyllithium, sec-butyllithium, phenyllithium or methyllithium. Preferably, n-butyllithium is used. In one embodiment a solution of n-butyllithium in hexanes is used. In another embodiment, 2-methyltetrahydrofurane or tetrahydrofurane can be added to the hexane solution of butyllithium. Usually, approximately an equimolar amount of butyllithium and compound of Formula II is used. A slight molar excess of the lithiating agent is possible, fox example of 1.2.

Commercially available butyllithium is delivered as a solution in hexanes. In a preferred embodiment hexane should be treated as an additional component in the solvent systems listed above. In a more preferred embodiment, where the solvent system for lithiation comprises tetrahydrofurane or 2-methyltetrahydrofurane and toluene, hexane introduced together with butyllithium constitutes an additional solvent.

The solutions of the reactants are cooled to temperatures below -20°C and the reactions are carried out under flow of inert gas.

The work up of the reaction mixture comprises quenching of the reaction mixture with methanol saturated with HCI. The obtained product of Formula V is subjected to extraction prior to the next step, i.e. the reduction.

In a second embodiment, the lithiation and the reaction with the gluconolactone IV is performed as a continuous flow process.

Preferably the lithiation and the following coupling are conducted in a nonpolar or medium polar environment. Suitable solvents include tetrahydrofurane, 2-methyltetrahydrofurane, dioxane, diethyl ether, dibutyl ether, diisopropyl ether, cyclopentyl methyl ether, methyl tert-butyl ether, toluene, pentane, hexane, and cyclohexane or mixtures thereof. Preferred mixtures comprise mixtures of tetrahydrofurane or 2-methyltetrahydrofurane with toluene or hexane or cyclohexane. In a preferred embodiment a mixture of tetrahydrofurane or 2-methyltetrahydrofurane with toluene is used, preferably a 1:3 mixture.

Lithiating agents which can be used are, for example, tert-butyllithium, sec-butyllithium, phenyllithium or methyllithium. Preferably, n-butyllithium is used as the lithiating agent. In one embodiment a solution of n-butyllithium in hexanes is used. In another embodiment, 2-methyltetrahydrofurane or tetrahydrofurane can be added to the hexane solution of butyllithium. Usually, approximately an equimolar amount of butyllithium and compound of Formula II is used. A slight molar excess of the lithiating agent is possible, fox example of 1.2.

Commercially available butyllithium is delivered as a solution in hexanes. In a preferred embodiment hexane should be treated as an additional component in the solvent systems listed above. In a more preferred embodiment, where the solvent system for lithiation comprises tetrahydrofurane or 2-methyltetrahydrofurane and toluene, hexane introduced together with butyllithium constitutes an additional solvent.

When the compound of Formula V is obtained in a flow process the lithiation is conducted in a first flow reactor at a temperature below 0, preferably at a temperature between 0 and -80°C, most preferably between -5°C and -40°C.

Then the reaction mixture with compound of Formula III and the solution of gluconolactone IV are introduced into a second flow reactor and reacted at a temperature below 0, preferably at a temperature between 0 and -80°C, more preferably between -10°C and -70°C and most preferably between -45°C and -65°C. Usually, an equimolar amount of gluconolactone IV with respect to the amount of compound of Formula II is used, however a slight molar excess of the gluconolactone IV is also possible, for example of 1.2.

By conducting the lithiation and the reaction with gluconolactone IV in a continuous flow process it was possible to increase the conversion of compound of Formula V to X=94% (as compared to X=80-85% for batch process) and consequently the yield of empagliflozin crude to Y=72% (as compared to Y=55% for batch process) mainly by reducing the amount of unreacted substrate of Formula II.

The work up of the reaction mixture obtained from the continuous flow process is conducted in the same manner as for the reaction mixture obtained in the batch process, namely the reaction mixture is quenched with methanol saturated with HCI. The obtained product of Formula V is subjected to extraction prior to the next step, i.e., the reduction.

**Table 1. Comparison of yield and amount of impurities (by UPLC) for different processes.**

| | Lithiation and coupling as batch processes and reduction without molecular sieves (not according to the invention) | Lithiation and coupling as batch processes and reduction in the presence of molecular sieves | Lithiation and coupling as a flow process and reduction without molecular sieves | Lithiation and coupling as a flow process and reduction in the presence of molecular sieves |
|---|---|---|---|---|
| **Yield** | 31.1 g (55%) | 5.0 g (53%) | 6.7 g (72.0%) | 6.6 g (70%) |
| **Impurity 1** | 0.24% | 0.17% | 0.16% | 0.13% |
| **Impurity 2** | 0.22% | 0.15% | 0.16% | 0.12% |
| **Total impurities** | 0.63% | 0.55% | 0.38% | 0.44% |

The optimal result was achieved when the lithiation performed as a flow process was combined with the use of molecular sieves in the reduction step. In this embodiment, the impurities 1 and 2 were only 0.13% and 0.12%, respectively, hence their amount was reduced by almost a half with respect to batch process without molecular sieves. Moreover, when flow chemistry was employed, the yield of the whole synthesis was increased, from 53% to 70%.

The present invention also provides a process for preparing a dosage form comprising empagliflozin, comprising the steps of preparing empagliflozin according to the invention and combining empagliflozin with one or more pharmaceutically acceptable excipients.

Empagliflozin is typically administered orally and so the dosage form is preferably an oral dosage form, most preferably a tablet. In such a case, the process further comprises a tableting step.

The oral dosage form may be a coated or uncoated tablet and may be prepared using standard techniques known in the art.

Where the oral dosage form is a coated tablet, the tablet is preferably a film-coated tablet. Typically, the film-coated tablet will contain 10 mg or 25 mg of empagliflozin. Excipients formulated with the active ingredient typically include lactose, magnesium stearate, and microcrystalline cellulose. Additionally, the film-coat is typically formulated with excipients which include hypromellose, talc, titanium dioxide, polyethylene glycol, and saccharin sodium.

Accordingly, the invention also provides empagliflozin obtainable by the process of the invention and an empagliflozin dosage form comprising empagliflozin obtainable by the process of the invention.

The products obtainable by the processes are different to the products obtainable by the processes of the prior art, as demonstrated by the data showing that empagliflozin has improved purity relative to the products obtained by the prior art. Indeed, empagliflozin is obtained with UPLC purity 99.90-100.0%.

The present invention will now be described with reference to the following examples, which are not intended to be limiting.

### Analytical methods

### Ultra performance liquid chromatography method

A liquid chromatograph with UV detector adjusted to ultra performance liquid chromatography was used.
Column: Acquity UPLC BEH Phenyl; 2.1 x 100 mm; particle size 1.7 µm
Detection at 224 nm
Mobile phase: A - 0.1% solution of ortho-phosphoric acid in water
Mobile phase: B - acetonitrile:methanol (40:60)
Gradient A:B from 65:35 to 0:100.

### Examples

### Example 1

### Preparation of methyl-C-(4-chloro-3-{4-[(3S)-tetrahydrofuran-3-yloxy]benzyl}phenyl)-α-D-glucopyranoside (Formula V) in a batch process.

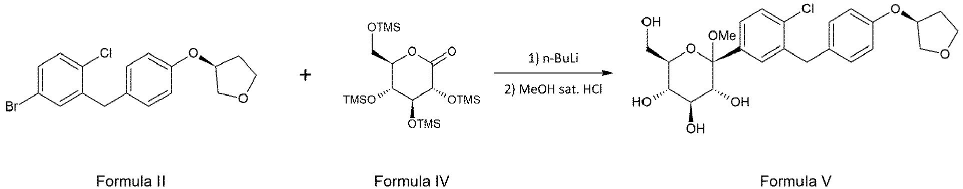

Compound of Formula II (61.2 g) was dissolved in a mixture of toluene (405 ml) and 2-methyltetrahydrofurane (135 ml) at ambient temperature. The solution was cooled down to a temperature below -30°C (T_{jacket} = -40°C) under flow of inert gas and subsequently a 2.5 M solution of butyllithium in hexanes (70 ml) was added dropwise within 10 min, the temperature was maintained in the range of -40°C to -28°C.

Simultaneously compound of Formula IV (77.7 g) was dissolved in toluene (324 ml) at ambient temperature. The solution was cooled down to a temperature below -30°C (T_{jacket} = -40°C) under flow of inert gas. Next, the cold solution of the lithium salt of compound of Formula II (T_{reaction} < -30°C) was added dropwise to the solution of compound of Formula IV keeping the internal temperature in the range of -40°C to -28°C. The reaction mixture was stirred for 15 min. Then methanol (240 ml) was added dropwise during 10 to 20 minutes at a temperature T_{reaction} ≤ -28°C (T_{jacket} ≤ -40°C) followed by stirring for 15 min at T_{reaction} ≤ -28°C. Then methanol saturated with HCI (5,8 - 6,2 g/100 ml) (160 ml) was added dropwise at a temperature of T_{reaction} ≤ -28°C (T_{jacket} ≤ -40°C) during 10 to 20 minutes. The reaction mixture was warmed up to T_{reaction} = 20-25°C during 30 to 60 minutes followed by stirring for 6 h at room temperature (UPLC 80-86% of compound of Formula V).

Next, the reaction mass was cooled down to ∼0°C and neutralized/basified with aqueous 4% Na₂CO₃ (about 210 ml) to a pH of ∼9.0-9.5 keeping internal temperature below 10°C. The reactor content was warmed up to room temperature and stirred for 30 min. After separation of layers, the aqueous phase was washed with dichloromethane (1 x 300 ml, 2 x 200 ml). Combined extracts were washed with 15% brine (200 ml) and concentrated under vacuum (starting from p=800 mbar, ending at p=50 mbar; T_{jacket} = 35-45°C) to give the product as a pale yellow/brown foam. Conversion: X=80-85% (UPLC purity UPLC=88-93%).

### Example 2

### Preparation of methyl-C-(4-chloro-3-{4-[(3S)-tetrahydrofuran-3-yloxy]benzyl}phenyl)-α-D-glucopyranoside (Formula V) in a flow reactor.

The flow reactor system was composed of three gear pumps, a stainless steel tubings and two sets of jacketed T-mixers followed by jacketed reactors. Steel tubings were immersed in an oil bath. Solutions were prepared as follows:
Solution A: (S)-3-[4-(5-bromo-2-chlorobenzyl)phenoxy]tetrahydrofuran (Formula II) (61.2 g, 0.166 mol) in toluene (275 ml) and 2-methyltetrahydrofurane (92 ml);
Solution B: n-butyllithium (1.6 M in hexanes, 110.0 ml, 0.176 mol) in 2-methyltetrahydrofurane (160 ml); Solution C: 2,3,4,6-tetra-O-trimethylsily-D-gluconolactone (Formula IV) (77.7g, 0.166 mol) in toluene (326 ml).

Solution A (9.5 ml/min) and solution B (6 ml/min) were transferred by steel tubings immersed in an oil bath having the temperature of T = -10-(-15°C) to the reactor 1 via T-mixer 1 to prepare the organolithium derivative of Formula III. Solution C (10 ml/min) and the solution with the organolithium derivative of Formula III (15.5 ml/min) were transferred by steel tubings immersed in oil bath having the temperature T = -50-(-55)°C to the reactor 2 via the T mixer 2. The total flow rate was 25.5 ml/min. The reaction mixture was then continuously quenched with MeOH saturated with HCI at a temperature of T = 0-5°C. After collecting the full batch, the reaction mixture was warmed up to room temperature and stirred for 6 h (UPLC 85-95% of compound of Formula V). The reaction was then cooled to T_{reaction} ≤ 5°C (T_{jacket} ≤ -10°C) and basified with 4% Na₂CO₃ (230 ml) added dropwise at T_{reaction} ≤ 5°C during 10 to 40 min. Next, the reaction was warmed to room temperature and the pH was controlled (pH = 9.0-9.5). The reaction was then stirred for 30 min and the layers were separated, the organic phase was utilized, the aqueous phase was collected with the product. Next, the compound of Formula V was extracted from the aqueous phase with dichloromethane (1 x 300 ml, 2 x 200 ml) and the combined organic layers were washed with 15% NaCl (200 ml). Combined extracts were washed with 15% brine (200 ml) and concentrated under vacuum (starting from p=800 mbar, ending at p=50 mbar; T_{jacket}=35-45°C) to give the product as a pale yellow/brown foam. Conversion: X=80-95% (UPLC purity UPLC=88-97%).

Reactor was evacuated to p=50 mbar at T_{jacket} = 35-45°C and refilled with inert gas, inertization was repeated. Next the residue was held for 2 h under p≤50 mbar at T_{jacket} = 35-45°C. Compound of Formula V (UPLC 89-93% purity) was isolated as a yellowish foam, then dissolved in a mixture of dichloromethane and acetonitrile and directed to the next step. Conversion X=90-94% (UPLC purity UPLC=90-97%).

### Example 3

### Preparation of crude empagliflozin according to the invention

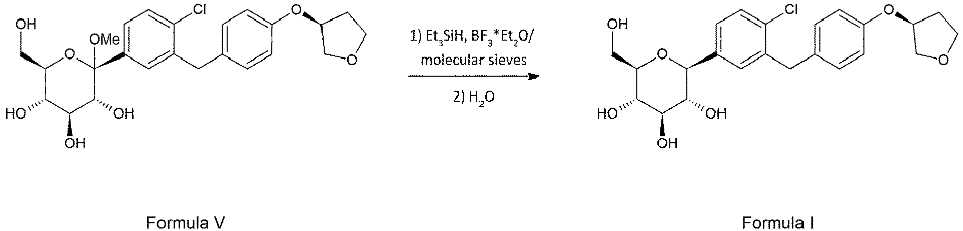

Dichloromethane (20 ml) and acetonitrile (40 ml) were added to the residue after distillation from Example 1 or 2 - equivalent of 10 g of compound of Formula V - and the reaction mixture was stirred at 20-50°C to full dissolution of the solid. Then the reaction mixture was cooled to 15-25°C and 1.0 g of activated molecular sieves 3 Å was added and the mixture was stirred for 60 min.

Acetonitrile (35 ml) and triethylsilane (7.92 g, 3.2 eq.) were loaded to second reactor. 1.0 g of activated molecular sieves 3 Å was added to the solution and the mixture was stirred for 60 min. Next, the content of the reactor was cooled down to the temperature of Treaction = -15°C (T_{jacket}=-17°C) under flow of inert gas. Boron trifluoride diethyl etherate (9.62 g; ≥46.5% BF₃ basis, 3.3 eq) was added dropwise (Treaction changed from ∼-16°C to ∼-10°C).

Next, the solution of compound of Formula V was added dropwise to the reactor with BF₃ diethyl etherate and triethylsilane (Treaction changed from ∼-15°C to ∼-5°C). The temperature was raised to T_{reaction} = 0-5°C (T_{jacket}=0-5°C) and the solution was stirred for 1 h. To the stirred mixture 120 ml of demi water were added. The reaction mixture was filtered to remove molecular sieves, cooled to T_{reaction} = 0-5°C and stirred at this temperature for 16 h. Next, the crude product was collected by filtration, washed on filter 2 times with a mixture of 10 ml of dichloromethane and 10 ml of acetonitrile, then with demi water (each portion 10 ml) until the pH of liquors was higher than 5.0. The product was dried in an oven drier at T = 80°C for 10 h. Empagliflozin (UPLC > 99.5% purity) was isolated as off-white solid 6.6 g (70% calculated on compound of Formula V).

### Example 4

### Purification of crude empagliflozin

2-propanol (10 x the mass of crude empagliflozin, 500 ml) and crude empagliflozin (50.0 g) were added to the reactor. Next, the content of the reactor was heated to reflux. The mixture was maintained at this temperature for 30 min in order to completely dissolve crude empagliflozin. The mixture was cooled to T = 20-25°C during 7 h and the suspension was left for 10 h. Next, the suspension of empagliflozin was filtered and the filtration cake was washed twice with 2-propanol (1 x mass of crude empagliflozin, 50 ml). The product was kept on filter for 1 h and directed to tray dryer for 2 h at T = 40-45°C, then 10-16 h at T = 90°C to obtain pure empagliflozin. Y=90-95%, 45.0-47.5 g, (UPLC empagliflozin > 99.5%).

### Comparative example

### Preparation of crude empagliflozin without molecular sieves

Dichloromethane (45 ml), acetonitrile (90 ml) and triethylsilane (32.9 g, 3.2 eq.) were loaded in a reactor and the mixture was stirred to dissolution of the solid. Next, the content of the reactor was cooled down to a temperature of T_{reaction} < -15°C (T_{jacket} = -17°C) under flow of inert gas and boron trifluoride diethyl etherate (40.5 g; ≥ 46.5% BF₃ basis, 3.3 eq) was added dropwise (T_{reaction} changed from ∼-16°C to ∼-10°C).

The second reactor was loaded with dichloromethane (75 ml), acetonitrile (150 ml) and 60.0 g of the residue after distillation containing compound of Formula V (from Example 1 or 2) obtained from 45.9 g (0.124 mol) of compound of Formula II and the mixture was stirred for 15 min to complete dissolution of the solid.

Next, the solution of compound of Formula V was added dropwise to the reactor with boron trifluoride diethyl etherate and triethylsilane (Treaction changed from ∼15°C to ∼5°C). Temperature was raised to T_{reaction} = 0-5°C (T_{jacket} = 0-5°C) and the mixture was stirred for 1h. To the stirred mixture 120 ml demi water were added during 5 min. The reaction mixture was cooled to Treaction = 0-5°C and stirred at this temperature for 16 h. Next, crude empagliflozin was collected by filtration, washed on filter 2 times with a mixture of 10 ml of dichloromethane and 10 ml of acetonitrile, then with demi water (each portion 10 ml) until the pH of liquors was higher than 5.0. The product was dried in oven drier at T = 80°C for 10 h. Empagliflozin (UPLC >99.0% purity) was isolated as off-white solid 31.1 g (55% calculated on compound of Formula II).

## Claims

1. Process for the preparation of empagliflozin comprising lithiation of (3S)-3-[4-(5-bromo-2-chlorobenzyl)phenoxy]tetrahydrofuran using lithium reagent, coupling the lithiated anion species with (3R,4S,5R,6R)-3,4,5-tris[(trimethylsilyl)oxy]-6-{[(trimethylsilyl)oxy]methyl}tetrahydro-2H-pyran-2-one and reducing the product methyl 1-C-(4-chloro-3-{4-[(3S)-tetrahydrofuran-3-yloxy]benzyl)phenyl)-α-D-glucopyranoside] using triethylsilane and boron trifluoride diethyl etherate and optionally purifying the crude product, wherein the reduction is conducted in the presence of molecular sieves.

2. Process according to claim 1, wherein the reduction is performed in dichloromethane, acetonitrile, tetrahyfrofurane, 2-methyltetrahydrofurane, tetrahydropyran, methyltetrahydropyran or mixtures thereof.

3. Process according to claim 2, wherein the reduction is performed in the solvent mixture dichloromethane and acetonitrile.

4. Process according to claim 3, wherein the ratio of dichloromethane to acetonitrile is 1:2 (v/v).

5. Process according to any of the previous claims, wherein the reduction is conducted at a temperature ranging from -30°C to 10°C.

6. Process according to any of the previous claims, wherein the molecular sieves are type 3 Å or 4 Å.

7. Process according to any of the previous claims, wherein the mass of molecular sieves used in the reduction constitutes 5-50% of the mass of methyl 1-C-(4-chloro-3-{4-[(3S)-tetrahydrofuran-3-yloxy]benzyl}phenyl)-α-D-glucopyranoside, preferably 5-20%.

8. Process for the preparation of empagliflozin according to any of the previous claims, wherein the lithiation of (3S)-3-[4-(5-bromo-2-chlorobenzyl)phenoxy]tetrahydrofuran using a lithium reagent is conducted in a flow process to form the lithiated anion species, and the coupling of the lithiated anion species with (3R,4S,5R,6R)-3,4,5-tris[(trimethylsilyl)oxy]-6-{[(trimethylsilyl)oxy]methyl}tetrahydro-2H-pyran-2-one to form a glycoside is conducted in a flow process.

9. Process according to any of the previous claims, wherein lithiation is conducted in tetrahydrofurane, 2-methyltetrahydrofurane, dioxane, diethyl ether, dibutyl ether, diisopropyl ether, cyclopentyl methyl ether, methyl tert-butyl ether, toluene, pentane, hexane, and cyclohexane or mixtures thereof.

10. Process according to any of the previous claims, wherein the lithiation is conducted in a mixture of tetrahydrofurane or 2-methyltetrahydrofurane with toluene or hexane or cyclohexane.

11. Process according to any of the previous claims, wherein the lithiation is conducted in mixture of tetrahydrofurane or 2-methyltetrahydrofurane with toluene, preferably a 1:3 (v/v) mixture.

12. Process according to any of the previous claims, wherein the lithiation agent is n-butyllithium, preferably as a solution in hexanes.

13. Process according to any of the previous claims, wherein the lithiation and the coupling reaction are conducted at a temperature ranging from 0°C to -80°C.

14. Process according to any of the previous claims, wherein the lithiation and the coupling reaction are conducted at a temperature ranging from -45°C to -65°C.
